(19) **Europäisches Patentamt**
**European Patent Office**
**Office européen des brevets**

(11) Publication number: **0 279 618 B1**

(12) # EUROPEAN PATENT SPECIFICATION

(45) Date of publication of patent specification :
**23.10.91 Bulletin 91/43**

(51) Int. Cl.⁵ : **C12N 11/00**

(21) Application number : **88301255.1**

(22) Date of filing : **16.02.88**

(54) **Improvements in probiotic-type products.**

(30) Priority : **20.02.87 GB 8704058**

(43) Date of publication of application :
**24.08.88 Bulletin 88/34**

(45) Publication of the grant of the patent :
**23.10.91 Bulletin 91/43**

(84) Designated Contracting States :
**AT BE CH DE ES FR GR IT LI NL SE**

(56) References cited :
**EP-A- 0 209 510**
**FR-A- 2 247 253**
**FR-A- 2 414 554**
**GB-A- 1 069 400**
**GB-A- 2 066 069**
**GB-A- 2 072 502**
**US-A- 3 984 575**

(73) Proprietor : **Porter, William Leslie**
**Animax Ltd. Gilray Road Vinces Road**
**Industrial Estate**
**Diss Norfolk (GB)**

(72) Inventor : **Porter, William Leslie**
**Animax Ltd. Gilray Road Vinces Road**
**Industrial Estate**
**Diss Norfolk (GB)**

(74) Representative : **Stone, Patrick**
**28 Edenside Drive**
**Attleborough Norfolk NR17 2EL (GB)**

EP 0 279 618 B1

## Description

This invention relates to a method of producing an animal feed supplement which improves the capacity of a foodstuff to produce weight gain in animals.

Known probiotic products for oral administration to animals, either specifically or by inclusion in feed, consist of fermented cultures of live non-pathogenic bacteria, typically lactobacilli and/or streptococci.

Probiotics are used to improve rate of growth and feed conversion efficiency in young animals and to reduce incidence of enteritis and diarrhoea. However, the effects of such products are highly variable.

The essential mode of action of probiotics is not, in fact, understood. It is commonly thought that they enhance the numbers of non-pathogenic bacteria in the gut, which may assist digestion, possibly through enzyme production, and also increase competition with populations of potentially harmful bacteria such as Escherichia coli. However, this is not proved. In fact, the normal dosage of a probiotic is usually insufficient to add appreciably to the intestinal population and this casts doubt on the commonly believed mode of action referred to.

Additionally, since the mode of action is not in fact sufficiently understood, meaningful quality control of probiotic products is virtually impossible.

From U.K. Patent Specification No. 1069400 is known a method of producing an animal feed supplement wherein a culture containing bacillus subtilis is allowed to ferment only to an early stage in the fermentation process, and the resulting fluid is then heat treated at a temperature of between 50 and 80 degrees Centigrade. The resulting product, when used as an animal feed supplement for chicks, results over a period of 5 weeks in a weight gain of up to 7 per cent over and above that of a basic diet.

A principal object of this invention is to provide an economical method of producing a more effective animal feed supplement which improves the capacity of a foodstuff to produce weight gain in animals. This can permit meaningful assay and hence meaningful quality control.

According to the present invention, there is provided a method of producing an animal feed supplement which improves the capacity of a foodstuff to produce weight gain in animals, comprising the steps of : preparing a base culture, sterilising the base culture, innoculating the base culture with an active starter culture in the form of non-pathogenic bacterial cells, and allowing the culture to ferment ; characterised in that the cells are isolated from the bulk of the fluid substrate, and in that the residue of cells is heated to not less than 100 degrees Centigrade for a period of time sufficient to kill the cells.

It will be understood that, conventionally, probiotic products are products containing live bacteria. The animal feed supplement produced in accordance with the present invention contains only killed bacteria, but is a product intended for oral administration in an analogous manner to some known probiotics.

The animal feed supplement produced in accordance with the present invention can take several forms. Thus, for example, the killed cells, in the form of a wet slurry, can have a bacteriostat added thereto for storage and oral administration in the wet state. Alternatively, the killed cells can be dried, optionally combined with a carrier, and included in an animal feed. Yet again, the killed cells can be absorbed in a drying agent, such as expanded mica, ready for inclusion in an animal feed.

Non-pathogenic bacterial cells which may be included in the liquid culture employed in this invention include lactobacilli and non-pathogenic streptococci, and also bacillus subtilis.

The animal feed supplement produced in accordance with the invention has been tested on chicks in trials lasting 14 days or more. Different groups of chicks were fed a basic diet as a control diet and other diets having allegedly beneficial additives, the additive for one group being the animal feed supplement produced in accordance with the invention. Trials were initially carried out using a commercial broiler feed as a control diet, to assess the effects of differing allegedly beneficial additives. Additives used were live bacterial probiotics known by the Trade Marks Pronifer® and Maxipro®. The growth and feed conversion efficiency of groups of chicks receiving these products were compared with similar groups of chicks receiving the same originally live bacterial products, but where the products had been subject to autoclaving so as to kill the bacterial cells present in the products. It was found that substantially improved results were obtained when the live bacterial probiotics were autoclaved, thus killing all bacteria present. Further trials were then carried out in which bacterial cultures were used as additives, both as whole cultures and after centrifugal separation into the bulk liquid substrate and the cellular concentrate or residue of killed bacteria. The results were compared against the same control diet as before and also the control diet with added killed yeast cells.

The statistical results of these trials have shown that, especially after a full trial of at least 14 days, the best liveweight gain and best feed conversion ratio is to be expected from the cellular concentrate or residue containing killed bacteria.

The cellular concentrate thus has, to an unexpected extent, nutritive benefit to the growth of the animals.

Quality control is enabled firstly because the cellular content of the bacterial culture may be determined in conventional manner before autoclaving, for example colony counting techniques using plate cultures, and secondly because an index of the content is readily obtained either by dry matter estimation of the culture or the concentrated slurry or by using acid or gas production as parameters of bacterial growth.

More generally, various advantages arising from use of the animal feed supplement produced in accordance with the invention are as follows : a) the total cell mass can be estimated and related to the effectiveness of the product ; b) stability is improved over conventional probiotics as no live bacteria are present ; c) the low volume of the cellular concentrate enables production of a solid, powdered or granular material, when this is required for inclusion in dry animal feeds ; d) liquid products can be manufactured using the killed bacterial cells, since the matter is inert (it is difficult to keep cells alive for long periods in a liquid) ; e) the liquid substrate is a by-product of the method of manufacture which, when used as a bulk liquid feed, is also found to possess useful nutritional properties.

A practical method of production of animal feed supplement in accordance with the invention will now be described by way of example.

First, one exemplary formulation of a base culture will be given :

| | |
|---|---|
| Milk powder | 60 g/l |
| Yeast extract | 4 g/l |
| Sucrose | 10 g/l |
| Beef and vegetable extracts | 1 g/l |
| Vitamin $B_{12}$ | 5 mg/l |
| Magnesium sulphate | 0.2 g/l |
| Manganous sulphate | 0.05 g/l |
| 'Tween 80'$^R$ | 1 m/l |

To this may be added :

| | |
|---|---|
| D. Potassium H. phosphate | 2.0 g/l |
| Sodium acetate | 5.0 g/l |
| Tri-ammonium citrate | 2.0 g/l, |

more especially to act as buffers if pH is not otherwise controlled during the subsequent fermentation process. The medium is made up to one litre with de-ionised water.

Production of the animal feed supplement is then carried out generally in accordance with the following steps :

1. The base culture is mixed using a shearing-type mixer.

2. The culture is sterilised by autoclaving at a pressure not less than 5 p.s.i., conveniently for 15 minutes at 121 degrees C. The culture could be sterilised in other known ways, if desired, which will ensure that any contaminants are killed.

3. The culture is cooled or allowed to cool to about 41 degrees C.

4. The base culture is inoculated with a small volume of an active starter culture, more especially but not exclusively a lactobacillus culture (such as Lactobacillus, fermentum) or a streptococci culture or bacillus subtilis.

5. The culture is allowed to ferment, at a temperature of about 41 degrees C, for a period of about 72 hours, whilst being subjected to gentle agitation.

6. During the fermentation step, a pH value of between 5 and 6, conveniently about 5.5, is maintained by means of NaOH and/or by means of the buffers previously referred to. If, as is preferred, pH is controlled by use of sodium hydroxide, this may be added initially, and then automatically, responsively to the output of a pH sensor.

7. During the fermentation process, gas production is monitored with a gas flowmeter, acid production is monitored by titration, and optionally cell production may be monitored by cell counting.

8. At the end of the fermentation period, the fermented culture is cooled or allowed to cool to ambient temperature Settling occurs during this period of cooling.

9. The supernatent liquid is then syphoned off. This leaves a residue in the form of a slurry containing the cells produced by fermentation.

10. The slurry residue is mixed by a shearing-type mixer.

11. The slurry is autoclaved at a pressure of at least 5 p.s.i., typically about 121 degrees C for 20 minutes. Autoclaving can be carried out at a higher pressure (and temperature) if desired. The result is a slurry with a high content of killed bacterial cells.

12. The killed cell slurry is again mixed by a shearing-type mixer.

13. The killed cell slurry is then dried, as by freeze drying or addition of a bacteriostat and mixing with a drying agent. Again, spray drying or other drying by use of heat may be employed instead.

14. The resulting killed cell residue in dried condition is combined with a carrier and constitutes an animal food supplement ready for use.

It is alternatively possible to produce a liquid product, again suitable for use as an animal feed supplement, by mixing the wet residue of step 12 or the dried residue of step 13 with a liquid carrier together with a bacteriostat, although a solid product is preferred for inclusion in dry feed.

A cellular concentrate containing only autoclaved cells of <u>Lactobacillus fermentum</u> and added to a basic control diet has been tested on broiler chicks over a period of 0 to 21 days of age with the following results. In the table, the column headings designate the number of parts by weight of cellular concentrate added to one million parts of weight of control feed, and the successive rows in the table indicate weight gain (WG) in grams, weight gain as a percentage of control (WG%), the feed conversion ratio (FCR) and the feed conversion ratio as a percentage (FCR%), taking the FCR for control as 100.

<u>Table</u>

|       | <u>0</u> | <u>50</u> | <u>100</u> | <u>200</u> | <u>400</u> |
|-------|------|--------|--------|--------|--------|
| WG    | 335.25 | 381.08 | 401.81 | 390.52 | 371.36 |
| WG%   | 100  | 113.67 | 119.85 | 116.49 | 110.77 |
| FCR   | 2.963 | 2.216 | 2.096 | ·2.139 | 2.541 |
| FCR%  | 100  | 74.79  | 70.74  | 72.19  | 85.75  |

A prototype commercial food additive produced substantially by the method hitherto described has been tested on broiler chicks over a period of 0 to 18 days. The control diet used for this test was a commercially available mix containing wheat (63.4), soya extract (25.0), full fat soya (3.6), dicalcium sulphate (2.0), DL-Methionine (0.22), salt (0.2), Minvite 204 (0.5) and soya bean oil (4.8), with added choline chloride (0.5), the figures being percentages.

In the following table, the column headings indicate grams/ton of additive employed, first when the fermented slurry residue was autoclaved and second when this residue was boiled.

<u>Table</u>

|      | Control | Autoclaved <u>Approx.30 g/tonne</u> | Boiled <u>Approx.30 g/tonne</u> |
|------|---------|-----------------------|-------------------|
| WG   | 360.17  | 381.75                | 376               |
| WG%  | 100     | 105.99                | 104.04            |

This result indicates that boiling is nearly as effective as autoclaving as a means of processing the bacterial cells.

It is also important to note that trials with broiler chicks have been conducted in which WG and FCR were compared when a) the cellular concentrate was used as a food additive, b) the bulk liquid substrate emerging as a by-product of the process was used as an additive, c) the bulk fermented fluid autoclaved but without separation of the bulk fluid substrate was used as an additive, and d) the bulk dried culture without separation and autoclaving was used as an additive. These trials have shown that a substantially improved WG and FCR are obtained with the cellular concentrate as compared with any of the other additives. It is therefore to be understood that process step 9 of the above-described method of production, in which the bulk liquid substrate is syphoned off or otherwise removed, is an essential step in production of the animal feed additive in accordance with the invention. Clearly, step 11 of the process is also essential.

The identity of the substance obtained from killed microbial cells and having considerable beneficial effect on animal growth and feed conversion efficiency is not yet known. However, tests for antibiotic activity have proved negative.

It will be clear that the animal feed supplement produced in accordance with the invention is useful for administration to animals other than chicks, such as pigs and other monogastrics, as well as ruminants.

## Claims

1. A method of producing an animal feed supplement which improves the capacity of a foodstuff to produce weight gain in animals, comprising the steps of : preparing a base culture, sterilising the base culture, innoculating the base culture with an active starter culture in the form of non-pathogenic bacterial cells, and allowing the culture to ferment ; characterised in that the cells are isolated from the bulk of the fluid substrate, and in that the residue of cells is heated to not less than 100 degrees Centigrade for a period of time sufficient to kill the cells.

2. A method according to claim 1, characterised in that the cells are killed by autoclaving at a pressure of at least 5 p.s.i. and a temperature of about 121 degrees Centigrade for about 20 minutes.

3. A method according to claim 1 or claim 2, characterised in that the bacterial cells are lactobacilli, non-pathogenic streptococci or bacillus subtilis.

4. A method according to claim 1 or claim 2 or claim 3, characterised in that the residue of killed cells is dried.

5. A method according to any of claims 1 to 4, characterised in that the cell residue is mixed with a carrier.

6. An animal food supplement when produced by the method of any of claims 1 to 5.

## Patentansprüche

1. Ein Verfahren zur Herstellung eines Tierfutterzusatzes, das die Fähigkeit eines Nahrungsmittels verbessert, um einen Gewichtszuwachs bei Tieren hervorzurufen, mit den Schritten : Bereitstellen einer Basiskultur, Sterilisieren der Basiskultur, Okulieren der Basiskultur mit einer aktiven Initialkultur in Form von nicht pathogenischen, bakteriellen Zellen und Gestatten der Kultur zu fermentieren ; dadurch **gekennzeichnet**, daß die Zellen aus der Masse des Fluidsubstrates isoliert werden, und daß der Zellenrest auf nicht weniger als 100°C für eine Zeitperiode erwärmt wird, die ausreicht, um die Zellen zu töten.

2. Ein Verfahren nach Anspruch 1, dadurch **gekennzeichnet**, daß die Zellen durch einen Aufenthalt im Autoklaven bei einem Druck von mindestens 5 p.s.i. und einer Temperatur von ungefähr 121° C ca. 20 Minuten lang getötet werden.

3. Ein Verfahren nach Anspruch 1 oder 2, dadurch **gekennzeichnet**, daß die bakteriellen Zellen Lactobazillen, nicht pathogenische Streptokokken oder Bacillus Subtilis sind.

4. Ein Verfahren nach Anspruch 1 oder 2 oder 3, dadurch **gekennzeichnet**, daß der Rest der getöteten Zellen getrocknet wird.

5. Ein Verfahren nach einem der Ansprüche 1 bis 4, dadurch **gekennzeichnet**, daß der Zellenrest mit einem Träger gemischt wird.

6. Ein Tierfutterzusatz, das nach dem Verfahren gemäß einem der Ansprüche 1 bis 5 hergestellt ist.

## Revendications

1. Une méthode de production d'un supplément à l'alimentation animale améliorant la capacité d'une ration alimentaire à produire un gain de poids chez les animaux, qui comprend les étapes suivantes :
— préparer une culture de base

— stériliser la culture de base

— inoculer la culture de base avec une culture de démarrage active sous forme de bactéries non pathogènes

— laisser la culture fermenter

Cette méthode est caractérisée par le fait que les cellules sont isolées de la masse de substrat fluide (surnageant) et que ce résidu cellulaire est chauffé à une température minimum de 100°C pendant une durée suffisante pour tuer les cellules.

2. Une méthode en conformité avec la revendication 1, caractérisée par le fait que les cellules sont tuées en autoclave à une pression de 5 PSI (0,33 bar) et à une température d'environ 121°C pendant 20 minutes.

3. Une méthode en conformité avec la revendication 1 ou la revendication 2, caractérisée par le fait que les bactéries sont des lactobacilles, des streptocoques non pathogènes ou des Bacillus subtilis.

4. Une méthode en conformité avec la revendication 1 ou la revendication 2 ou la revendication 3, caractérisée par le fait que le résidu de cellules tuées est séché.

5. Une méthode en conformité avec n'importe laquelle des revendications 1 à 4, caractérisée par le fait que le résidu cellulaire est mélangé à un support.

6. Un supplément à l'alimentation animale produit selon la méthode de n'importe laquelle des revendications 1 à 5.